# EUROPEAN PATENT APPLICATION

(11) **EP 4 494 568 A1**
(43) Date of publication of application: **22.01.2025**
(21) Application number: 23195049.4
(22) Date of filing: 04.09.2023
(51) Int. Cl.: A61B 8/08, A61B 8/00

(54) **DYNAMIC MEDICAL IMAGING DURATION WARNING**

(30) Priority: 17.07.2023 US 202363527236 P
(71) Applicant: Koninklijke Philips N.V., 5656 AG Eindhoven (NL)
(72) Inventor: FINCKE, Jonathan, Eindhoven (NL); LEE, Hyeon Woo, Eindhoven (NL); GHANI, Muhammad Usman, 5656 AG Eindhoven (NL); RAJU, Iyyavu Balasundar, Eindhoven (NL)
(74) Representative: Philips Intellectual Property & Standards

(57) **Abstract**

An ultrasound system includes a processor configured to cause the ultrasound system to: detect (S325) when the ultrasound system starts a scan for an ultrasound examination; obtain (S380) a plurality of ultrasound images; track (S345) an amount of time the ultrasound system is scanning once the scan starts; compare (S360) a tracked amount of time to a predetermined threshold; and display (S390) the tracked amount of time on a display overlaid on each of the plurality of ultrasound images.

## Description

### FIELD OF THE INVENTION

The present disclosure relates generally to ultrasound imaging. In particular to systems and methods for tracking ultrasound imaging progress.

### BACKGROUND OF THE INVENTION

The use of ultrasound in assessments of patients with traumatic injuries is becoming commonplace along the continuum of care. Ultrasound may be used at the start of care for traumatic injuries when emergency medical services (EMS) become involved, and may be used all the way to an intensive care unit (ICU). In some cases, the users of the ultrasound scanners in trauma scenes have minimal training. In some cases, potential users avoid ultrasound because they are not confident or properly trained on how to utilize ultrasound to help diagnose their patients. These users need digital aids to help them scan more safely and effectively.

In cases of using ultrasound to assess patients with traumatic injuries, ultrasound scans must be performed quickly to allow the provision of adequate care to the patient. Rapid care delivery is particularly important when the Focused Assessment with Sonography for Trauma (FAST) exam is used on unstable or severely injured patients. Untrained or new ultrasound users may spend too much time acquiring FAST exam imagery, delaying care which could potentially be detrimental to the patient's health. Delays in care could be life threatening in some cases.

In the case of using ultrasound to assess patients with traumatic injuries, ultrasound scans must be done quickly to allow the provision of adequate care to the patient. Lesser experienced users of ultrasound may become distracted while balancing patient care and scanning.

Because the user base of ultrasound is expected to grow rapidly, particular for users involved with trauma care, and because the credentialing and training will be less thorough relative to professional sonographers, ultrasound systems and devices will need features to support lesser trained, untrained and/or new users.

### SUMMARY OF THE INVENTION

It is inter alia an object of this invention to assist users in performing ultrasound scans, particularly FAST scans. For this purpose, the invention proposes features to indicate and/or alert users of how long they have spent using the ultrasound systems and devices. For example, by using an overall system or device time indicator or an indicator per scan.

The invention is defined by the independent claims. The dependent claims define advantageous embodiments.

According to an embodiment of the present disclosure, an ultrasound system includes a memory that stores instructions and a processor that executes the instructions. When executed by the processor, the instructions cause the ultrasound system to: detect when the ultrasound system starts a scan for an ultrasound examination; obtain or receive a plurality of ultrasound images; track an amount of time the ultrasound system is scanning once the scan starts; compare a tracked amount of time to a predetermined threshold; and display the plurality of ultrasound images with the tracked amount of time on a display overlaid on each of the plurality of ultrasound images.

In an example, the ultrasound system further comprises:
an ultrasound cart comprising:
   the memory, and
   the processor; and
a display configured to display the plurality of ultrasound images with the tracked amount of time overlaid on each of the plurality of ultrasound images.

In an example, the ultrasound system further comprises:
a portable transducer comprising:
   the memory, and
   the processor,
wherein the portable transducer is configured to link to an external device for the ultrasound examination via an application installed on the external device, and
wherein the plurality of ultrasound images with the tracked amount of time overlaid on each of the plurality of ultrasound images are displayed on the external device.

In an example, the ultrasound examination comprises a focused assessment with sonography for trauma (FAST) ultrasound examination.

In an example, when executed by the processor, the instructions further cause the ultrasound system to:
display a warning when the tracked amount of time exceeds the predetermined threshold.

In an example, the ultrasound system, when executed by the processor, the instructions further cause the ultrasound system to:
accept the predetermined threshold as a variable input set by a user.

In an example, when executed by the processor, the instructions further cause the ultrasound system to:
detect when an ultrasound probe is placed on a body of a subject of the ultrasound examination; and
identify a part of the body being scanned; and
detect that the ultrasound system starts the scan for the ultrasound examination based on detecting when the ultrasound probe is placed on the body of the subject.

In an example, when executed by the processor, the instructions further cause the ultrasound system to:
apply a trained machine learning model to detect when the ultrasound probe is placed on the body of the subject and to identify a part of the body being scanned; and
reset the amount of time being tracked when the part of the body being scanned is changed.

In an example, when executed by the processor, the instructions further cause the ultrasound system to:
detect activation of a button; and
detect that the ultrasound system starts the scan for the ultrasound examination based on detecting the activation of the button.

According to another embodiment of the present disclosure, a method for ultrasound imaging includes detecting, by a controller comprising a memory that stores instructions and a processor that executes the instructions, when an ultrasound system starts a scan for an ultrasound examination; obtaining or receiving a plurality of ultrasound images; tracking an amount of time the ultrasound system is scanning once the scan starts; comparing a tracked amount of time to a predetermined threshold; and displaying the plurality of ultrasound images with the tracked amount of time on a display overlaid on each of the plurality of ultrasound images.

In an example, the method further comprises:
displaying a warning when the tracked amount of time exceeds the predetermined threshold.

In an example, the method further comprises:
accepting the predetermined threshold as a variable input set by a user.

In an example, the method further comprises:
detecting when an ultrasound probe is placed on a body of a subject of the ultrasound examination; and
identifying a part of the body being scanned; and
detecting that the ultrasound system starts the scan for the ultrasound examination based on detecting when the ultrasound probe is placed on the body of the subject.

In an example, the method further comprises:
applying a trained machine learning model to detect when the ultrasound probe is placed on the body of the subject and to identify a part of the body being scanned; and
resetting the amount of time being tracked when the part of the body being scanned is changed.

In an example, the method further comprises:
detecting activation of a button; and
detecting that the ultrasound system starts the scan for the ultrasound examination based on detecting the activation of the button.

According to another embodiment of the present disclosure there is provided a computer program product such as a tangible non-transitory computer-readable storage medium that stores a computer program. When executed by a processor, the computer program causes the processor or a system comprising said processor to: detect when the ultrasound system starts a scan for an ultrasound examination; obtain a plurality of ultrasound images; track an amount of time the ultrasound system is scanning once the scan starts; compare a tracked amount of time to a predetermined threshold; and display the tracked amount of time on a display the plurality of ultrasound images with overlaid on each of the plurality of ultrasound images.

In an example, when executed by the processor, the instructions further cause the processors to:
detect when an ultrasound probe is placed on a body of a subject of the ultrasound examination;
identify a part of the body being scanned;
   detect that the ultrasound system starts the scan for the ultrasound examination based on detecting when the ultrasound probe is placed on the body of the subject;
apply a trained machine learning model to detect when the ultrasound probe is placed on the body of the subject and to identify a part of the body being scanned; and
reset the amount of time being tracked when the part of the body being scanned is changed.

According to yet another embodiment of the present disclosure, a tangible, non-transitory computer-readable medium stores instructions. When executed by a processor, the instructions cause an ultrasound system to: detect when the ultrasound system starts a scan for an ultrasound examination; obtain a plurality of ultrasound images; track an amount of time the ultrasound system is scanning once the scan starts; compare a tracked amount of time to a predetermined threshold; and display the tracked amount of time on a display overlaid on each of the plurality of ultrasound images.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 illustrates a system for dynamic medical imaging duration warning, in accordance with a representative embodiment.
Fig. 2 illustrates another system for dynamic medical imaging duration warning, in accordance with a representative embodiment.
Fig. 3 illustrates a method for dynamic medical imaging duration warning, in accordance with a representative embodiment.
Fig. 4 illustrates a user interface progression for dynamic medical imaging duration warning, in accordance with a representative embodiment.
Fig. 5 illustrates a user interface showing detected subject matter for dynamic medical imaging duration warning, in accordance with a representative embodiment.
Fig. 6 illustrates a computer system, on which a method for dynamic medical imaging duration warning is implemented, in accordance with another representative embodiment.

### DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, ultrasound devices and systems may be adapted to help new users such as when performing the FAST exam so as to alert users as to how long they have spent using the ultrasound equipment. The teachings herein are applicable to at least ultrasound devices and systems used at point of care for trauma and similar scenarios. Ultrasound users and managers of ultrasound users may be enabled to set timing limits that will trigger on screen warnings when the user has exceeded a pre-established scan time limit, and the timing limits may be varied for different scenarios. As a result, the likelihood of ultrasound being used too long when inexperienced users are scanning patients who are in need of urgent medical attention may be reduced by automatically timing how long the user is scanning and by displaying the scan time on the ultrasound system user interface.

The teachings herein relating to point of care ultrasound may be used in a variety of contexts, including for untrained or relatively untrained users of ultrasound, expert users of ultrasound, users learning ultrasound, emergency departments, paramedics and cross-trained firefighter/paramedics, military medical care, mass casualty events, and users of increasingly-common portable ultrasound systems.

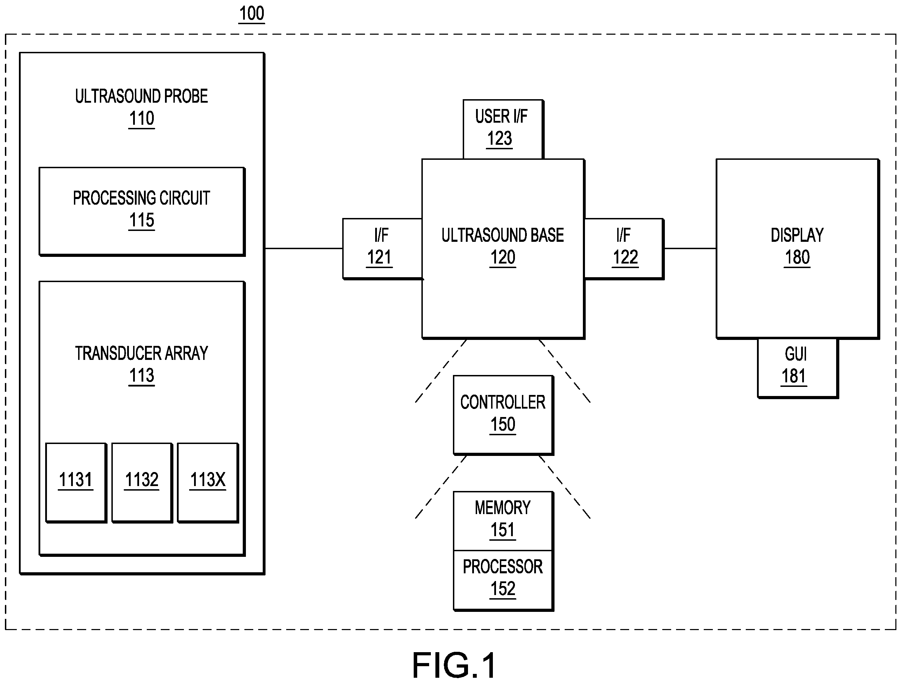

Fig. 1 illustrates a system 100 for dynamic medical imaging duration warning, in accordance with a representative embodiment.

The system 100 in Fig. 1 is a system for dynamic medical imaging duration warning and includes components that may be provided together or that may be distributed. The system 100 includes an ultrasound probe 110, an ultrasound base 120, and a display 180.

The ultrasound probe 110 includes a processing circuit 115 and a transducer array 113. The processing circuit 115 may comprise a memory for storing data and instructions and a application-specific integrated circuit (ASIC) and/or a processor for processing data and instructions. The transducer array 113 includes an array of transducer elements including at least a first transducer element 1131, a second transducer element 1132, and an Xth transducer element 113X. That is, the ultrasound probe 110 comprises an ultrasound transducer and associated electronics to produce images that are then rendered on the display 180 for the user to view. The ultrasound probe 110 includes the transducer array 113, and the transducer array 113 converts electrical energy into sound waves which bounce off of body tissue, and receives echoes of the sound waves and converts the echoes into electrical energy. The transducer array 113 may include dozens, hundreds or thousands of individual transducer elements. The ultrasound probe 110 may transmit a beam to produce images and may detect the echoes. The processing circuit 115 may process ultrasound images captured by the transducer array 113 of the ultrasound probe 110.

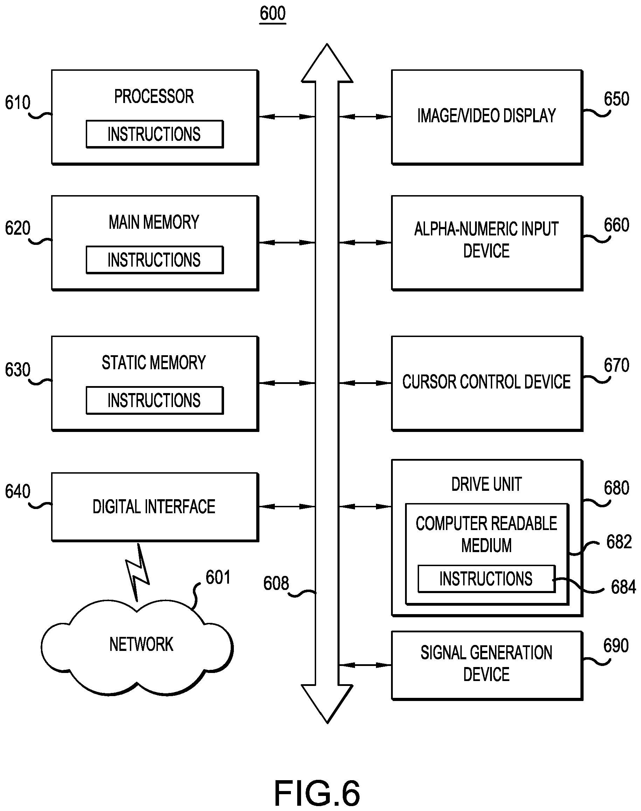

The ultrasound base 120 includes a first interface 121, a second interface 122, a third interface 123, and a controller 150. A computer that can be used to implement the ultrasound base 120 is depicted in Fig. 6, though an ultrasound base 120 may include more or fewer elements than depicted in Fig. 1 or Fig. 6. One or more of the interfaces may include ports, disk drives, wireless antennas, or other types of receiver circuitry that connect the controller 150 to other electronic elements. The first interface 121 connects the ultrasound base 120 to the ultrasound probe 110, and may comprise a port, an antenna, and/or another type of physical component for wired or wireless communications. The second interface 1220 connects the ultrasound base 120 to the display 180, and may also comprise a port, an antenna, and/or another type of physical component for wired or wireless communications. The third interface 123 is a user interface, and may comprise buttons, keys, a mouse, a microphone, a speaker, switches, a touchscreen or other type of display separate from the display 180, and/or other types of physical components that allow a user to interact with the ultrasound base 120 such as to enter instructions and receive output.

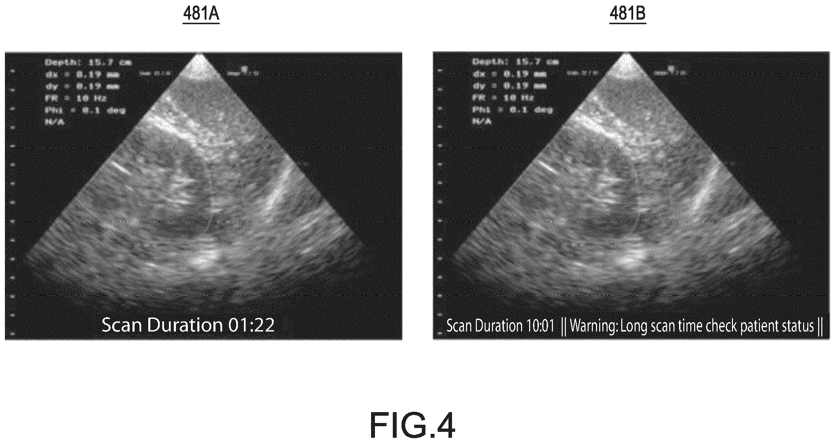

The controller 150 includes at least a memory 151 that stores instructions and a processor 152 that executes the instructions. The instructions stored in the memory 151 may comprise a software program for generating an alarm on or for the system 100. In the system 100, the alarm may be a visual and/or audible alarm provided via a user interface and generated by the instructions stored in the memory 151. For example, an alarm may comprise a warning displayed on the display 180. The software program in the memory 151 may generate contextual warnings based on the use of the system 100. The software program in the memory 151 may automatically detect when and where a user has started scanning. An algorithm included in the software program may initiate the start and stop of a timer which is presented to the user via the display 180. Such a timer is shown in Fig. 4 on the left, with a warning when the scan is taking too long on the right. The algorithm may use signal processing in some embodiments, learning techniques in other embodiments, and/or may be based on user inputs to the system 100. User inputs may include, for example, gain and depth adjustment. In some cases, users may activate a button to initiate the start of scan timing.

The display 180 may be local to the ultrasound base 120 or may be remotely connected to the ultrasound base 120, such as wirelessly. The display 180 may be connected to the ultrasound base 120 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users.

The controller 150 may perform some of the operations described herein directly and may implement other operations described herein indirectly. For example, the controller 150 may indirectly control operations such as by generating and transmitting content to be displayed on the display 180. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

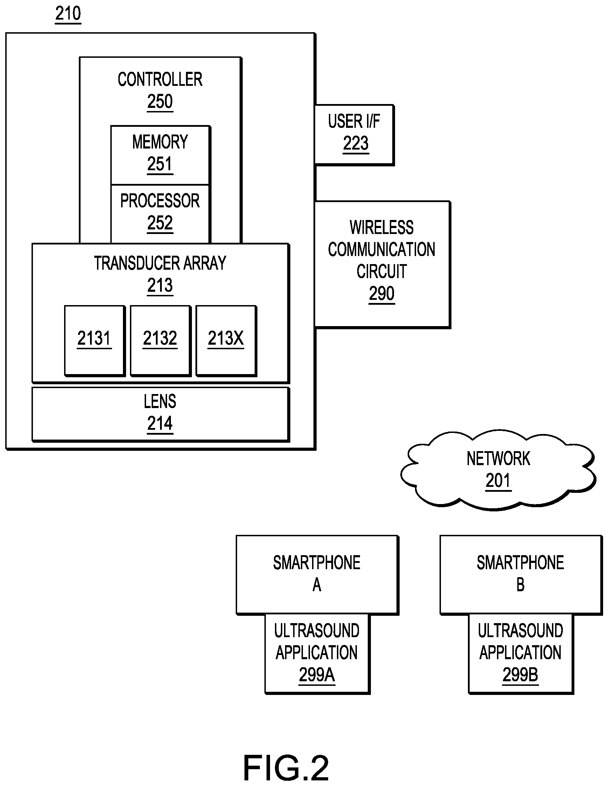

Fig. 2 illustrates another system for dynamic medical imaging duration warning, in accordance with a representative embodiment.

In Fig. 2, an ultrasound probe 210 and a smartphone A and a smartphone B are connected over a network 201.

The network 201 may comprise a local wireless network such as a WiFi network, though the network 201 may also or alternatively include wired elements such as wires connected to smartphone A and smartphone B via USB cables.

The ultrasound probe 210 includes a transducer array 213, a lens 214, a user interface 223, a controller 250 and a wireless communication circuit 290. The transducer array 213 includes at least a first transducer element 2131, a second transducer element 2132, and an Xth transducer element 213X. The transducer array 213 may correspond to the transducer array 113 and may comprise an array of individually controllable transducer elements. The transducer array 213 converts electrical energy into sound waves which bounce off of body tissue, and receives echoes of the sound waves and converts the echoes into electrical energy. The transducer array 213 may include dozens, hundreds or thousands of individual transducer elements. The ultrasound probe 210 may transmit a beam to produce images and may detect the echoes. The lens 214 may be used to transmit ultrasound beams and to receive echoes of ultrasound beams. The user interface 223 may be used by a user to interact with the ultrasound probe 210. The wireless communication circuit 290 may be used to communicate with smartphone A and smartphone B via the network 201. That is, the ultrasound probe 210 comprises an ultrasound transducer and associated electronics to produce images that are then rendered on displays of smartphone A and/or smartphone B.

Smartphone A stores and executes an ultrasound application 299A. Smartphone B stores and executes an ultrasound application 299B. The ultrasound application 299A and the ultrasound application 299B may be configured to enable smartphone A and smartphone B interact with the ultrasound probe 210 via the network 201. For example, ultrasound application 299A and ultrasound application 299B may be configured to enable displays of ultrasound images from the ultrasound probe 210. Ultrasound application 299A and ultrasound application 299B may also be configured to overlay warnings on the displays of ultrasound images from the ultrasound probe 210. In some embodiments, determinations of whether a warning should be made may be performed by the ultrasound application 299A and the ultrasound application 299B. For example, the ultrasound application 299A and the ultrasound application 299B may each comprise a model that can be applied to dynamic information as described below.

The controller 250 includes at least a memory 251 that stores instructions and a processor 252 that executes the instructions. The instructions stored in the memory 251 may comprise a software program with a model such as an artificial intelligence model, as well as the same or a different software program for generating a user interface on an ultrasound device. In Fig. 2, the user interface may be generated by the instructions stored in the memory 251 and may be displayed on a display of the smartphone A or smartphone B. The software program(s) in the memory 251 generate warnings when a tracked amount of time for an ultrasound procedure is greater than a threshold. The warning may be displayed on displays of the smartphone A and/or the smartphone B. The software program in the memory 251 may generate contextual warnings based on the use of the ultrasound probe 210. The software program in the memory 251 may automatically detect when and where a user has started scanning. An algorithm included in the software program may initiate the start and stop of a timer which is presented to the user via a display of smartphone A or smartphone B. Such a timer is shown in Fig. 4 on the left, with a warning when the scan is taking too long on the right. The algorithm may use signal processing in some embodiments, learning techniques in other embodiments, and/or may be based on user inputs to either the ultrasound probe 210 or to smartphone A or smartphone B. User inputs may include, for example, gain and depth adjustment. In some cases, users may activate a button to initiate the start of scan timing.

The controller 250 may perform some of the operations described herein directly and may implement other operations described herein indirectly. For example, the controller 250 may indirectly control operations such as by generating and transmitting content to be displayed on a display of the smartphone A or smartphone B. The controller 250 may directly control other operations such as logical operations performed by the processor 252 executing instructions from the memory 251 based on input received from electronic elements and/or users via the interfaces. Accordingly, the processes implemented by the controller 250 when the processor 252 executes instructions from the memory 251 may include steps not directly performed by the controller 250.

Ultrasound systems of types such as the system 100 in Fig. 1 include an ultrasound probe and an ultrasound base, and are designed for use by physicians and sonographers who have received extensive training. Mobile ultrasound systems such as the combination of the ultrasound probe 210 and smartphone A and smartphone B in Fig. 2 are much more likely to be usable and used by a wider range of users who will not have in depth training, as they expect such ultrasound devices and systems to function more like sensors than as imaging tools. Therefore, new ultrasound devices require features that automate aspects of ultrasound scanning or prevent the user from misusing the technology mistakenly. An example of when a user may misuse ultrasound is when the user takes too long to perform a FAST exam. No simple way was previously provided to indicate to the user how long they have been scanning other than tracking themselves with a clock in the vicinity of the ultrasound examination. Additionally, lesser trained users or users who do the FAST examinations less frequently may not recall what an appropriate amount of time is to complete a FAST exam during the resuscitation of patient.

The system 100 and the ultrasound probe 210 combined with ultrasound application 299A and/or ultrasound application 299B automatically time how long the user has been scanning and display the time on a user interface such as the display 180 or a screen of the ultrasound probe 210 or smartphone A and/or smartphone B. The user interfaces showing the expired time and any appropriate warning provide a heads-up display of scan time to the physician. This also allows the manager of a user to set timing limits that will trigger on screen warnings that the user has exceeded a pre-established scan time. Software programs stored in the memory 151 of the controller 150 or the memory 251 of the controller 250 may detect when the probe is on the body and what part of the body to start timing once the scan has begun. In some embodiments, a simpler set of features may be used such as when the preset has been selected or the system 100 or the ultrasound probe 210 has been turned on.

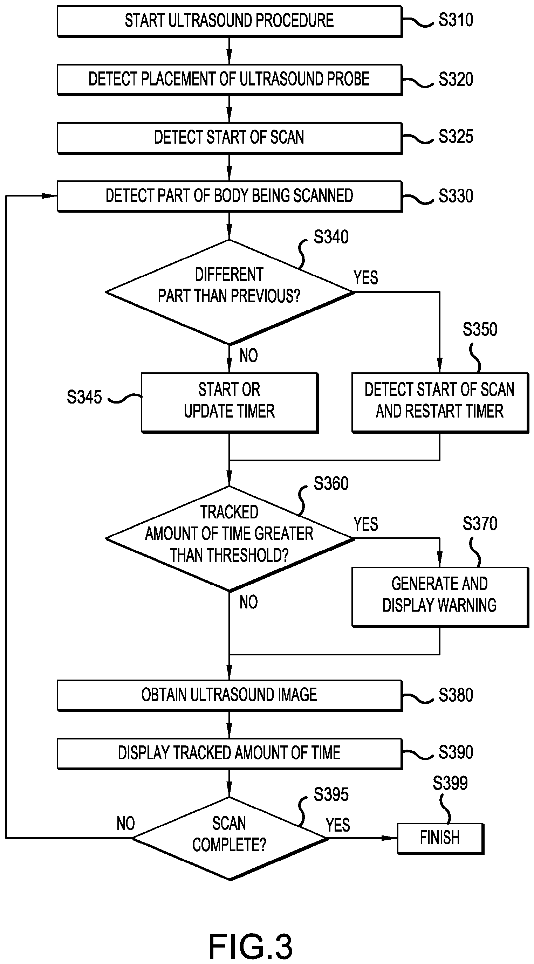

Fig. 3 illustrates a method for dynamic medical imaging duration warning, in accordance with a representative embodiment.

The method of Fig. 3 may be performed by the system 100 including the controller 150 in Fig. 1, by the ultrasound probe 210 in Fig. 2, or by a combination of the ultrasound probe 210 and one or both of smartphone A and/or smartphone B in Fig. 2.

At S310, an ultrasound procedure is started. The ultrasound procedure may be started at S310 by turning on or otherwise activating or initiating the ultrasound probe 110 and ultrasound base 120 in Fig. 1 or the ultrasound probe 210 and one or both of smartphone A and/or smartphone B in Fig. 2. The ultrasound procedure may comprise an ultrasound examination such as a focused assessment with sonography for ultrasound (FAST) ultrasound examination. The ultrasound probe 110, the ultrasound base 120 or the ultrasound probe 210 may detect activation of a button, and detect that the ultrasound system starts the scan for the ultrasound examination based on detecting the activation of the button.
At S320, placement of the ultrasound probe is detected. Placement of the ultrasound probe may be detected by a contact sensor, such as a capacitance touch sensor, a piezo touch sensor, or a resistance touch sensor. The contact sensor may be provided at or proximate to the bottom of the ultrasound probe 110 in Fig. 1 or the ultrasound probe 210 in Fig. 2.

At S325, a start of a scan is detected. The start of the scan may be detected by movement of the ultrasound probe or when substantive content consistent with an ultrasound scan is detected based on pixel data generated by the transducer array 113 or the transducer array 213.

At S330, a part of the body being scanned is detected. An image analysis program may be applied to pixel data generated by the transducer array 113 or the transducer array 213, and may detect the part of the body based on shape patterns of intensity in the pixel data, or another type of pattern. For example, the detection at S330 may detect whether a lung or liver is being scanned.

At S340, a determination is made as to whether the part of the body being scanned is the same as a previous part of the body being scanned, if the determination was previously made in the same ultrasound procedure. If the part of the body is the same part of the body previously being scanned (S340 = No), at S345 the timer is updated. If the determination at S340 is the first time the determination is being made in the current ultrasound procedure, the timer is started at S345. If the part of the body being scanned is different than a part of the body previously being scanned in the same ultrasound procedure (S340 = Yes), at S350 the start of a new scan is detected and the timer is restarted. That is, S350 may reflect an assumption that the sonographer is not stuck scanning a body part, so that detection that a new body part is being scanned may be assumed to warrant a restart of the timer. In some embodiments, the determinations at S340 may be more complex, such as when the part of the body being scanned is a particular body part, and other types of available information indicate that the start of a new scan at S350 should be overruled. For example, some particular types of injuries may simply not allow for restarting a timer, so that the determination at S340 is skipped and only the overall time from the start of the first scan is tracked.

At S360, a determination is made as to whether a tracked amount of time is greater than a threshold. The threshold may be fixed for all body parts, or may vary based on the type of body part and/or based on demographic information of the patient or other types of information such as experience level of the sonographer.

If the tracked amount of time is not greater than the threshold (S360 = No), at S380 an ultrasound image is obtained. If the tracked amount of time is greater than the threshold (S360 = Yes), at S370 a warning is generated and displayed and then provided with the ultrasound image at S380. The warning may also or alternatively be audibly output.

At S390, a tracked amount of time is displayed. The display may show the tracked amount of time in minutes and seconds, for example. The tracked amount of time may be displayed on a display overlaid on each of a plurality of ultrasound images. When the ultrasound base 120 is an ultrasound cart, the display 180 may display the ultrasound image with the tracked amount of time overlaid on the ultrasound images. When the ultrasound probe 210 is a portable transducer and is configured to link to an external device, the ultrasound image with the tracked amount of time overlaid on the ultrasound image may be displayed on the external device such as the smartphone A or the smartphone B. The display of the tracked amount of time may be varied such as by font, size and/or color based on how much the current ultrasound scan is over a threshold.

At S395, a determination is made as to whether the scan is complete. If the scan is complete (S395 = Yes), the process finishes at S399. The scan may be deemed complete when power to the ultrasound probe 110 or the ultrasound probe 210 is turned off, or when the ultrasound probe 110 or the ultrasound probe 210 does not capture imagery of anatomy for a predetermined amount of time such as 5 seconds. If the scan is not complete (S395 = No), the process returns to S330.

Fig. 4 illustrates a user interface progression for dynamic medical imaging duration warning, in accordance with a representative embodiment.

In Fig. 4, a first user interface 481A and a second user interface 481B show ultrasound images from different times in an ultrasound examination. The first user interface 481A and the second user interface 481B each show the user how long a scan has been performed. In some embodiments, a warning may be displayed to the user if the scan exceeds a predetermined threshold. The predetermined threshold may be provided as a variable input set by a user and accepted by the system 100 and by the ultrasound probe 210. Thresholds may vary based on the subject matter of the ultrasound examination or other contextual factors.

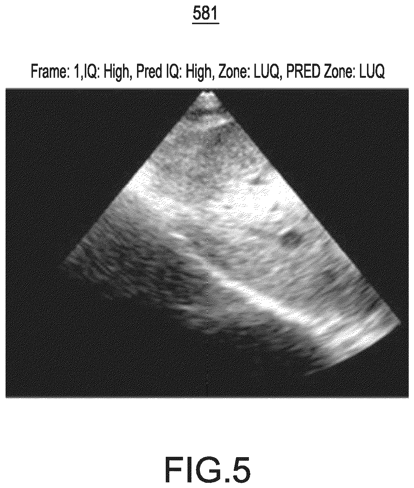

Fig. 5 illustrates a user interface showing detected subject matter for dynamic medical imaging duration warning, in accordance with a representative embodiment.

Fig. 5 shows a user interface 581. An example of an image on the user interface 581 results from applying a trained machine learning model to what an ultrasound probe is being used to image. In Fig. 5, the trained machine learning model has detected proper coupling of an ultrasound probe such as the ultrasound probe 110 or the ultrasound probe 210, as well as the anatomical zone the user is scanning. In some embodiments, trained machine learning models such as artificial intelligence algorithms are used to detect when the user has the ultrasound probe on the body of the subject and what anatomical zone the user is scanning. A timer may start when contact between the ultrasound probe and the body is detected and may end when the ultrasound probe is no longer in contact with the body and/or when a change in the scanning zone is detected. Each anatomical zone may be timed independently. The user or a manager of the user may configure time limits for the entire scan and/or for individual zones of an ultrasound examination such as a FAST examination.

Fig. 6 illustrates a computer system, on which a method for dynamic medical imaging duration warning is implemented, in accordance with another representative embodiment.

Referring to Fig. 6, the computer system 600 includes a set of software instructions that can be executed to cause the computer system 600 to perform any of the methods or computer-based functions disclosed herein. The computer system 600 may operate as a standalone device or may be connected, for example, using a network 601, to other computer systems or peripheral devices. In embodiments, a computer system 600 performs logical processing based on digital signals received via an analog-to-digital converter.

The computer program product may be software comprising the instructions, available for download from a server, e.g., via the internet. Alternatively, the instructions may be stored on a suitable (non-transitory) computer-readable medium such as an optical storage medium or a solid-state medium, which may or may not be supplied together with or as part of other hardware.

In a networked deployment, the computer system 600 operates in the capacity of a server or as a client user computer in a server-client user network environment, or as a peer computer system in a peer-to-peer (or distributed) network environment. The computer system 600 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 600 can be incorporated as or in a device that in turn is in an integrated system that includes additional devices. In an embodiment, the computer system 600 can be implemented using electronic devices that provide voice, video or data communication. Further, while the computer system 600 is illustrated in the singular, the term "system" shall also be taken to include any collection of systems or sub-systems that individually or jointly execute a set, or multiple sets, of software instructions to perform one or more computer functions.

As illustrated in Fig. 6, the computer system 600 includes a processor 610. The processor 610 may be considered a representative example of a processor of a controller and executes instructions to implement some or all aspects of methods and processes described herein. The processor 610 is an article of manufacture and/or a machine component. The processor 610 is configured to execute software instructions to perform functions as described in the various embodiments herein. The processor 610 may be a general-purpose processor or may be part of an application specific integrated circuit (ASIC). The processor 610 may also be a microprocessor, a microcomputer, a processor chip, a controller, a microcontroller, a digital signal processor (DSP), a state machine, or a programmable logic device. The processor 610 may also be a logical circuit, including a programmable gate array (PGA), such as a field programmable gate array (FPGA), or another type of circuit that includes discrete gate and/or transistor logic. The processor 610 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 600 further includes a main memory 620 and a static memory 630, where memories in the computer system 600 communicate with each other and the processor 610 via a bus 608. Either or both of the main memory 620 and the static memory 630 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 620 and the static memory 630 are articles of manufacture and/or machine components. The main memory 620 and the static memory 630 are computer-readable mediums from which data and executable software instructions can be read by a computer (e.g., the processor 610). Each of the main memory 620 and the static memory 630 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 600 further includes a video display unit 650, such as a liquid crystal display (LCD), an organic light emitting diode (OLED), a flat panel display, a solid-state display, or a cathode ray tube (CRT), for example. Additionally, the computer system 600 includes an input device 660, such as a keyboard/virtual keyboard or touch-sensitive input screen or speech input with speech recognition, and a cursor control device 670, such as a mouse or touch-sensitive input screen or pad. The computer system 600 also optionally includes a disk drive unit 680, a signal generation device 690, such as a speaker or remote control, and/or a network interface device 640.

In an embodiment, as depicted in Fig. 6, the disk drive unit 680 includes a computer-readable medium 682 in which one or more sets of software instructions 684 (software) are embedded. The sets of software instructions 684 are read from the computer-readable medium 682 to be executed by the processor 610. Further, the software instructions 684, when executed by the processor 610, perform one or more steps of the methods and processes as described herein. In an embodiment, the software instructions 684 reside all or in part within the main memory 620, the static memory 630 and/or the processor 610 during execution by the computer system 600. Further, the computer-readable medium 682 may include software instructions 684 or receive and execute software instructions 684 responsive to a propagated signal, so that a device connected to a network 601 communicates voice, video or data over the network 601. The software instructions 684 may be transmitted or received over the network 601 via the network interface device 640.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, dynamic medical imaging duration warning enables ultrasound devices and systems to alert uses as to how long they have spent using the ultrasound devices and systems, such as when performing the FAST exam. The teachings herein are applicable to ultrasound devices and systems used at point of care for trauma and similar scenarios. Ultrasound users and managers of ultrasound users may be enabled to set timing limits that will trigger on screen warnings that the user has exceeded a pre-established scan time limit, and the timing limits may be varied for different scenarios. As a result, the likelihood of ultrasound devices and systems from being used too long when inexperienced users are scanning patients who are in need of urgent medical attention may be reduced by automatically timing how long the user is scanning and by displaying the scan time on the ultrasound system user interface.

Although dynamic medical imaging duration warning has been described with reference to several exemplary embodiments, it is understood that the words that have been used are words of description and illustration, rather than words of limitation. Changes may be made within the purview of the appended claims, as presently stated and as amended, without departing from the scope of dynamic medical imaging duration warning in its aspects. Although dynamic medical imaging duration warning has been described with reference to particular means, materials and embodiments, dynamic medical imaging duration warning is not intended to be limited to the particulars disclosed; rather dynamic medical imaging duration warning extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

The reference signs in the claims shall not be construed as limiting the claims. Measures recited in mutually dependent claims may advantageously be used in combination.

## Claims

1. An ultrasound system (100), comprising:
a processor (152, 252) configured to:
detect (S325) when the ultrasound system starts a scan for an ultrasound examination;
obtain (S380) a plurality of ultrasound images;
track (S345) an amount of time the ultrasound system is scanning once the scan starts;
compare (S360) a tracked amount of time to a predetermined threshold; and
display (S390), on a display, the plurality of ultrasound images with the tracked amount of time overlaid on each of the plurality of ultrasound images.

2. The ultrasound system of claim 1, further comprising:
an ultrasound cart (120) comprising the processor; and
a display (180) configured to display the plurality of ultrasound images with the tracked amount of time overlaid on each of the plurality of ultrasound images.

3. The ultrasound system of claim 1, further comprising:
a portable transducer (213) comprising the processor, wherein the portable transducer is configured to link to an external device for the ultrasound examination via an application installed on the external device comprising the display.

4. The ultrasound system of any one of the preceding claims, wherein the processor is further configured to:
display (S370) a warning when the tracked amount of time exceeds the predetermined threshold.

5. The ultrasound system of any one of the preceding claims, wherein the processor is further configured to:
detect (S320) when an ultrasound probe is placed on a body of a subject of the ultrasound examination; and
identify (S330) a part of the body being scanned; and
detect (S325) that the ultrasound system starts the scan for the ultrasound examination based on detecting when the ultrasound probe is placed on the body of the subject.

6. The ultrasound system of claim 5, wherein the processor is further configured to:
apply a trained machine learning model to detect when the ultrasound probe is placed on the body of the subject and to identify a part of the body being scanned; and
reset the amount of time being tracked when the part of the body being scanned is changed.

7. A computer-implemented method for ultrasound imaging, the method comprising:
detecting (S325) when an ultrasound system starts a scan for an ultrasound examination;
receiving (S380) a plurality of ultrasound images;
tracking (S345) an amount of time the ultrasound system is scanning once the scan starts;
comparing (S360) a tracked amount of time to a predetermined threshold; and
displaying (S390) on a display the plurality of ultrasound images with the tracked amount of time overlaid on each of the plurality of ultrasound images.

8. The method of claim 7, further comprising:
generating (S370) a warning when the tracked amount of time exceeds the predetermined threshold, wherein optionally the warning is displayed on a display.

9. The method of claim 7 or 8, further comprising:
receiving the predetermined threshold as a variable input set by a user.

10. The method of any one of claims 7 to 9, further comprising:
detecting (S320) when an ultrasound probe is placed on a body of a subject of the ultrasound examination; and
identifying (S330) a part of the body being scanned; and
detecting (S325) that the ultrasound system starts the scan for the ultrasound examination based on detecting when the ultrasound probe is placed on the body of the subject.

11. The method of claim 10, further comprising:
applying a trained machine learning model to detect when the ultrasound probe is placed on the body of the subject and to identify a part of the body being scanned; and
resetting the amount of time being tracked when the part of the body being scanned is changed.

12. A computer program product that includes instructions, which when executed by a processor, cause the processor to carry out the method of any one of claims 7 to 11.
